(19) Europäisches Patentamt
European Patent Office
Office européen des brevets

(11) Publication number: **0 317 314 B1**

(12) # EUROPEAN PATENT SPECIFICATION

(45) Date of publication of patent specification: **04.11.92**

(51) Int. Cl.$^5$: **A61K 7/06**

(21) Application number: **88310858.1**

(22) Date of filing: **17.11.88**

The file contains technical information submitted after the application was filed and not included in this specification

(54) **Shampoo compositions.**

(30) Priority: **19.11.87 US 122986**

(43) Date of publication of application:
**24.05.89 Bulletin 89/21**

(45) Publication of the grant of the patent:
**04.11.92 Bulletin 92/45**

(84) Designated Contracting States:
**AT BE CH DE FR GB GR IT LI LU NL SE**

(56) References cited:
**EP-A- 0 034 846**
**GB-A- 759 199**

**CHEMICAL ABSTRACTS, vol. 81, no. 10, 9th September 1974, page 315, abstract 54333b, Columbus, Ohio, US; & JP-A-74 12 041 (NONOGAWA MITSUO) 02-02-1974**

**MARTINDALE, THE EXTRA PHARMACOPOEIA, page 502, selenium sulphide, The Pharmaceutical Press, London, GB**

(73) Proprietor: **THE PROCTER & GAMBLE COMPANY**
**One Procter & Gamble Plaza**
**Cincinnati Ohio 45202(US)**

(72) Inventor: **McCall, Patrick Columnkille**
**3992 Creek Road**
**Cincinnati Ohio 45241(US)**
Inventor: **Cothran, Philip Earl**
**6941 Rob Vern Drive**
**Cincinnati Ohio 45239(US)**

(74) Representative: **Brooks, Maxim Courtney et al**
**Procter & Gamble (NTC) Limited Whitley Road Longbenton**
**Newcastle-upon-Tyne NE12 9TS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 317 314 B1

## Description

The present invention is related to antidandruff shampoos containing selenium sulfide which shampoos achieve dispersion of the selenium sulfide particles through the use of low levels of specified cellulosic polymers.

Lotion shampoos are widely accepted due to their ease of use, including spreading the shampoo through the hair. However, when particulate, small size active ingredients are used in lotion shampoos, dispersion of those ingredients such as selenium sulfide presents a variety of problems.

The prior art discloses antidandruff shampoos containing components designed to suspend particulate matter. Japanese Application with Open for Public Inspection No. 60,810, May 19, 1977 (Lion Fat & Oil), discloses shampoos containing 5% to 50% of an anionic surfactant, 1% to 10% of a fatty acid diethanol amide, 0.1% to 10% of an insoluble fine powder and 1% to 10% of an ethylene glycol ester. US-A-4,470,982, to Winkler discloses similar compositions containing from 11% to 20% of an anionic surfactant, from 4% to 6% of a suspending agent, from 2% to 4% of an amide, a particulate antidandruff agent and water. GB-A-1,051,268, December 14, 1966 to Colgate-Palmolive Company discloses selenium sulfide shampoos containing suspending agents.

While these references disclose suspending antidandruff actives, they do not provide satisfactory answers to the problems associated with dispersing particulate antidandruff agents.

It has been surprisingly found by the present inventors that certain selenium sulfide compositions can utilize low levels of specified polymeric materials to disperse the selenium sulfide particles.

It is an object of the present invention, therefore, to provide stable selenium sulfide lotion shampoos.

It is a further object of the present invention to provide selenium sulfide lotion shampoos utilizing low levels of nonionic polymeric dispersing agents.

It is a still further object of the present invention to provide methods of shampooing hair with improved selenium sulfide compositions.

These and other objects will become readily apparent from the detailed description which follows.

Unless otherwise indicated, all percentages and ratios herein are by weight. Additionally, all measurements are made at 25° C. in the composition or on the pure material unless otherwise specified.

The present invention relates to a lotion shampoo composition comprising:

(a) from 10% to 30% by weight of a synthetic surfactant;

(b) from 0.1% to 5.0% of selenium sulfide having an average particle size of less than 25 µm;

(c) from 0.001% to 0.1% of a cellulose polymer, selected from hydroxypropyl methyl cellulose, carboxymethyl cellulose, sodium carboxymethyl cellulose, hydroxyethyl cellulose and mixtures thereof; and

(d) water.

The essential components of the present invention as well as optional components are given in the following paragraphs.

### Surfactant

An essential component of the present compositions is a surfactant. The surfactant, which may be selected from any of a wide variety of synthetic anionic, amphoteric, zwitterionic and nonionic surfactants, is present at a level of from 10% to 30%, preferably from 15% to 22%, most preferably from 18% to 20%.

Synthetic anionic surfactants can be exemplified by the alkali metal salts of organic sulfuric reaction products having in their molecular structure an alkyl radical containing from 8 - 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$ - $C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and other known in the art.

Zwitterionic surfactants can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from 8 to 18

carbon atoms and one contains an anionic water-solubilizing group, e.g., carboxy, sulfonate, sulfate, phosphate, or phosphonate. A general formula for these compounds is:

$$R^2 - \overset{(R^3)_x}{\overset{|}{Y^{(+)}}} - CH_2 - R^4 - Z^{(-)}$$

wherein $R^2$ contains an alkyl, alkenyl, or hydroxy alkyl radical of from 8 to 18 carbon atoms, from 0 to 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to about 3 carbon atoms; x is 1 when Y is a sulfur atom and 2 when Y is a nitrogen or phosphorus atom; $R^4$ is an alkylene or hydroxyalkylene of from 1 to 4 carbon atoms and Z is a radical selected from the group consisting of carboxylate, sulfonate, sulfate, phosphonate, and phosphate groups.

Examples include:
4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P, P-diethyl-P-3,6,9-trioxatetradexocylphosphonio]-2-hydroxy-propane-I -phosphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N-di(2-hydroxyethyl)-N-(2-hydroxydodecyl)ammonio]-butane-1-carboxylate;
3-[S-ethyl-S-(3-dodecoxy-2-hydroxypropyl)sulfonio]-propane-1-phosphate;
3-[P,P-dimethyl-P-dodecylphosphonio]-propane-1-phosphonate; and
5-[N,N-di(3-hydroxypropyl)-N-hexadecylammonio]-2-hydroxy-pentane-1-sulfate.

Other zwitterionics such as betaines are also useful in the present invention. Examples of betaines useful herein include the high alkyl betaines such as coco dimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl) carboxy methyl betaine, stearyl bis-(2-hydroxy-propyl) carboxymethyl betaine, oleyl dimethyl gamma-carboxypropyl betaine, lauryl bis-(2-hydroxypropyl) and alpha-carboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxy-ethyl) and sulfopropyl betaine, amido betaines and amidosulfobetaines, wherein the $RCONH(CH_2)_3$ radical is attached to the nitrogen atom of the betaine are also useful in this invention.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from 8 to 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, N-alkyltaurines such as the one prepared by reacting dodecylamine with sodium isethionate according to the teaching of US-A-2,658,072, N-higher alkyl aspartic acids such as those produced according to the teaching of US-A-2,438,091, and the products sold under the trade name Miranol (RTM) and described in US-A-2,528,378.

Nonionic surfactants, which are preferably used in combination with an anionic, amphoteric or zwitterionic surfactant, can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from about 40% to about 80% polyoxyethylene by weight and having a molecular weight of from 5,000 to 11,000 resulting from the reaction of ethylene oxide groups with a hydrophobic base

constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration, with ethylene oxide, e.g., a coconut alcohol ethylene oxide condensate having from 10 to 30 moles of ethylene oxide per mole of coconut alcohol, the coconut alcohol fraction having from 10 to 14 carbon atoms.

4. Long chain tertiary amine oxides corresponding to the following general formula:

$$R_1 R_2 R_3 N \rightarrow O$$

wherein $R_1$ contains an alkyl, alkenyl or monohydroxy alkyl radical of from about 8 to about 18 carbon atoms, from 0 to 10 ethylene oxide moieties, and from 0 to 1 glyceryl moiety, and $R^2$ and $R^3$ contain from 1 to 3 carbon atoms and from 0 to 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyldi-(2-hydroxyethyl) amine oxide, dimethyloctylamine oxide, dimethyl-decylamine oxide, dimethyltetradecylamine oxide, 3,6,9-trioxaheptadecyldiethylamine oxide, di(2-hydroxyethyl)-tetradecylamine oxide, 2-dode-coxyethyldimethylamine oxide, 3-dodecoxy-2-hydroxypropyldi(3-hydroxypropyl)amine oxide, dimethylhexadecylamine oxide.

5. Long chain tertiary phosphine oxides corresponding to the following general formula:

$$RR'R''P \rightarrow O$$

wherein R contains an alkyl, alkenyl or monohydroxyalkyl radical ranging from 8 to 18 carbon atoms in chain length, from 0 to 10 ethylene oxide moieties and from 0 to 1 glyceryl moiety and R' and R" are each alkyl or monohydroxyalkyl groups containing from 1 to 3 carbon atoms. The arrow in the formula is a conventional representation of a semipolar bond. Examples of suitable phosphine oxides are: dodecyldimethylphosphine oxide, tetradecyldimethylphosphine oxide, tetradecylmethylethylphosphine oxide, 3,6,9,-trioxaoctadecyldimethylphosphine oxide, cetyldimethylphosphine oxide, 3-dodecoxy-2-hydroxypropyldi(2-hydroxyethyl) phosphine oxide, stearyldimethylphosphine oxide, cetylethylpropylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi-(2-hydroxyethyl)phosphine oxide, tetradecylmethyl-2-hydroxypropylphosphine oxide, oleyldimethylphosphine oxide, 2-hydroxydodecyldimethylphosphine oxide.

6. Long chain dialkyl sulfoxides containing one short chain alkyl or hydroxy alkyl radical of 1 to 3 carbon atoms (usually methyl) and one long hydrophobic chain which contain alkyl, alkenyl, hydroxy alkyl, or keto alkyl radicals containing from 8 to 20 carbon atoms, from 0 to 10 ethylene oxide moieties and from 0 to 1 glyceryl moiety. Examples include: octadecyl methyl sulfoxide, 2-ketotridecyl methyl sulfoxide, 3,6,9,-trioxaoctadecyl 2-hydroxyethyl sulfoxide, dodecyl methyl sulfoxide, oleyl 3-hydroxypropyl sulfoxide, tetradecyl methyl sulfoxide, 3-methoxytridecyl methyl sulfoxide, 3-hydroxytridecyl methyl sulfoxide, 3-hydroxy-4-dodecoxybutyl methyl sulfoxide.

Many additional nonsoap surfactants are described in McCUTCHEON'S, DETERGENTS AND EMULSIFIERS, 1986 ANNUAL, published by Allured Publishing Corporation.

The above-mentioned surfactants can be used alone or in combination in the shampoo compositions of the present invention. The anionic surfactants, particularly the alkyl sulfates, the ethoxylated alkyl sulfates and mixtures thereof are preferred for use herein as well as the isethionates.

## Cellulosic Polymeric Material

Another essential component of the present compositions is a polymer material which is used at a low level to aid in keeping the particles of selenium sulfide dispersed. The polymer is a cellulosic material selected from hydroxypropyl methyl cellulose, carboxymethyl cellulose, hydroxyethyl cellulose, sodium carboxymethyl cellulose and mixtures thereof. The polymers are discussed in detail in Industrial Gums, Edited by Roy L. Whistler, Academic Press, Inc., (1973) and Handbook of Water-Soluble Gums and Resins, Edited by Robert L. Davidson, McGraw-Hill, Inc. (1980).

The polymer is used at a level of from 0.001% to 0.100%, preferably from 0.002% to 0.05%. Hydroxypropyl methyl cellulose is the preferred polymer.

Selenium Sulfide

Selenium sulfide is a staple item of commerce. Selenium sulfide is generally regarded as a compound having one mole of selenium and two moles of sulfur. However, it may take the form of a cyclic structure, $Se_xS_y$, wherein $x + y = 8$.

Selenium sulfide as provided by suppliers can be used in the present compositions provided the particle size of the selenium sulfide particles, on an average, is less than 25 µm preferably less than 15 µm. These measurements are made using a forward laser light scattering device (e.g., a Malvern 3600 instrument).

Selenium sulfide is present in the compositions of this invention at a level of from 0.1% to 5.0%, preferably from .6% to 2.5%.

Water

Water is the last essential component of the present invention. It is generally present at a level of from 20% to 95%, preferably from 60% to 85%.

Optional Components

A preferred optional component is a suspending agent. The suspending agent useful in the present compositions can, for example, be any of several long chain acyl derivative materials or mixtures of such materials. Included are ethylene glycol esters of fatty acids having from 16 to 22 carbon atoms. Preferred are the ethylene glycol stearates, both mono and distearate, but particularly the distearate containing less than about 7% of the mono stearate. Still other suspending agents found useful are alkanol amides of fatty acids, having from 16 to 22 carbon atoms, preferably 16 to 18 carbon atoms. Preferred alkanol amides are stearic monoethanolamide, stearic diethanolamide and stearic monoisopropanolamide. Still other long chain acyl derivatives include long chain esters of long chain fatty acids (e.g., stearyl stearate, cetyl palmitate, etc.); glyceryl esters (e.g., glyceryl distearate) and long chain esters of long chain alkanol amides (e.g., stearamide DEA distearate, stearamide MEA stearate).

Still other suitable suspending agents are alkyl ($C_{16-22}$) dimethyl amine oxides such as stearyl dimethyl amine oxide. If the compositions contain an amine oxide or a long chain acyl derivative as a surfactant the suspending function could also be provided and additional suspending agent may not be needed if the level of those materials are at least the minimum level given below.

Another suspending agent is veegum (RTM) magnesium, aluminum silicate). Xanthan gum is another agent used to suspend the selenium sulfide in the present compositions. This biosynthetic gum material is commercially available and is a heteropolysaccharide with a molecular weight of greater than 1 million. It is believed to contain D-glucose, D-mannose and D-glucuronate in the molar ratio of 2.8:2.0:2.0. The polysaccharide is partially acetylated with 4.7% acetyl. This information and other is found in Whistler, Roy L. Editor Industrial Gums - Polysaccharides and Their Derivatives New York: Academic Press, 1973. Kelco, a Division of Merck & Co., Inc. offers xanthan gum as Keltrol (RTM).

The suspending agent is preferably present at a level of from 0.10% to 5.0%, more preferably from 0.5% to 3.0%. The suspending agent serves to assist in suspending the selenium sulfide and may give pearlescence to the product. Mixtures of suspending agents are also suitable for use in the compositions of this invention.

Another preferred optional component for use in the present compositions is an amide. The amide used in the present compositions can be any of the alkanolamides of fatty acids known for use in shampoos. These are generally mono- and diethanolamides of fatty acids having from 8 to 14 carbon atoms. Preferred are coconut monoethanolamide, lauric diethanolamide and mixtures thereof.

The amide is present at a level of from 1% to 10%.

Another suitable optional component useful in the present compositions is a nonvolatile silicone fluid.

The nonvolatile silicone fluid may be either a polyalkyl siloxane, a polyaryl siloxane, a polyalkylaryl siloxane or a polyether siloxane copolymer and is preferably present at a level of from 0.1% to 10.00%, more preferably from 0.5% to 5.0%. Mixtures of these fluids may also be used and are preferred in certain executions. The dispersed silicone particles should also be insoluble in the shampoo matrix. This is the meaning of "insoluble" as used hereinbefore and hereinafter.

The essentially nonvolatile polyalkyl siloxane fluids that may be used include, for example, polydimethyl siloxanes with viscosities ranging from 5 to 600,000 $mm^2.s^{-1}$ (centistokes) at 25°C. These siloxanes are available, for example, from the General Electric Company as the Viscasil series and from Dow Corning as

the Dow Corning 200 series. The viscosity can be measured by means of a glass capillary viscometer as set forth in Dow Corning Corporate Test Method CTM0004, July 20, 1970. Preferably the viscosity range from 350 $mm^2.s^{-1}$ (centistokes) to 100,000 $mm^2.s^{-1}$ (centistokes).

The essentially nonvolatile polyalkylaryl siloxane fluids that may be used include, for example, polymethylphenylsiloxanes having viscosities of 15 to 30,000 $mm^2.s^{-1}$ (centistokes) at 25°C. These siloxanes are available, for example, from the General Electric Company as SF 1075 methyl phenyl fluoride or from Dow Corning as 556 Cosmetic Grade Fluid.

The essentially nonvolatile polyether siloxane copolymer that may be used is, for example, a polypropylene oxide modified dimethylpolysiloxane (e.g., Dow Corning DC-1248) although ethylene oxide or mixtures of ethylene oxide and propylene oxide may also be used.

References disclosing suitable silicone fluids include the previously mentioned US-A-2,826,551 to Geen; US-A-3,964,5000, June 22, 1976 to Drakoff; US-A-4,364,837 to Pader and GB-A-849,433 to Woolston. Silicon Compounds distributed by Petrarch Systems, Inc., 1984 also provides a very good listing of suitable silicone materials.

Another silicone material found especially useful in the present compositions to provide good dry combing is a silicone gum. Silicone gums described by Petrarch and others including US-A-4,152,416, May 1, 1979 to Spitzer, et al. and Noll, Walter, Chemistry and Technology of Silicones, New York: Academic Press 1968. Also describing silicone gums are General Electric Silicone Rubber Product Data Sheets Se 30, SE 33, SE 54 and SE 76. "Silicone gum" materials denote high moleculate weight polydiorganosiloxanes having a mass molecular weight of from about 200,000 to about 1,000,000. Specific examples include polydimethylsiloxane, (polydimethylsiloxane) (methylvinylsiloxane) copolymer, poly(dimethylsiloxane) (diphenyl) (methylvinylsiloxane) copolymer and mixtures thereof.

The shampoos herein can contain a variety of other non-essential optional components suitable for rendering such compositions more acceptable. Such conventional optional ingredients are well known to those skilled in the art, e.g., preservatives such as benzyl alcohol, methyl paraben, propyl paraben and imidazolidinyl urea; cationic surfactants such as cetyl trimethyl ammonium chloride, lauryl trimethyl ammonium chloride, tricetyl methyl ammonium chloride, stearyldimethyl benzyl ammonium chloride, and di-(partially hydrogenated tallow) dimethylammonium chloride; menthol; thickeners and viscosity modifiers such as block polymers of ethylene oxide and propylene oxide such as Pluronic (RTM) F88 offered by BASF Wyandotte, sodium chloride, sodium sulfate, propylene glycol, and ethyl alcohol; pH adjusting agents such as citric acid, succinic acid, phosphoric acid, sodium hydroxide, sodium carbonate, etc.; perfumes; dyes; and, sequestering agents such as disodium ethylenediamine tetraacetate. Such agents generally are used individually at a level of from 0.01% to 10%, preferably from 0.5% to 5.0% by weight of the composition.

The pH of the present compositions is generally not critical and may be obtained through the proper selection of surfactants or through the use of appropriate buffer systems to control pH such as citric acid/sodium citrate. Improved color stability is, however, achieved by maintaining the pH within the range of from about 2 to about 6, preferably from about 3 to about 5.

One method for manufacturing the present composition is described below in the Examples.

The present compositions are used in a conventional manner for cleaning hair. From 0.1g to 10g of a composition is applied to hair that has been wetted, generally with water, worked through the hair and then rinsed out.

The following Examples further describe and demonstrate the preferred embodiments within the scope of the present invention.

## EXAMPLES I - III

The following compositions are representative of the present invention.

| Component | Weight % I | II | III |
|---|---|---|---|
| EGDS Premix | | | |
| Water | 52.44 | 52.44 | 52.44 |
| Alkyl sulfate | 28.84 | 28.84 | 28.84 |
| Alkyl xylene sulfonate | 2.69 | 2.69 | 2.69 |
| Cetyl alcohol | 0.57 | 0.57 | 0.57 |
| Stearyl alcohol | 0.42 | 0.42 | 0.42 |
| Coconut monoethanolamide | 6.87 | 6.87 | 6.87 |
| Ethylene glycol distearate (EGDS) | 6.87 | 6.87 | 6.87 |
| Tricetyl methyl ammonium chloride | 1.30 | 1.30 | 1.30 |
| | 100.00 | 100.00 | 100.00 |
| Silicone Premix | | | |
| Alkyl (ethoxy) sulfate | 62.24 | 62.24 | 62.24 |
| Cetyl alcohol | 5.46 | 5.46 | 5.46 |
| Dimethicone (fluid/gum) | 30.30 | 30.30 | 30.30 |
| | 100.00 | 100.00 | 100.00 |
| Selenium Sulfide Premix | | | |
| DRO water | 63.74 | 68.24 | 63.74 |
| Sodium citrate | 0.95 | 0.95 | 0.95 |
| Citric acid | 0.81 | 0.81 | 0.81 |
| Sodium alkyl sulfate | 4.00 | 4.00 | 4.00 |
| Selenium sulfide | 25.00 | 25.00 | 25.00 |
| Preservative | 0.50 | 0.50 | 0.50 |
| Methocel Premix | 5.00 | 0.50 | 5.00 |
| | 100.00 | 100.00 | 100.000 |

| | | | |
|---|---|---|---|
| Methocel Premix | | | |
| Methocel (Hydroypropyl methyl cellulose) | 10.00 | 10.00 | 10.00 |
| DRO water | 90.00 | 90.00 | 90.00 |
| | 100.00 | 100.00 | 100.00 |
| Main Mix | | | |
| ECDS premix | 42.88 | 42.88 | 42.107 |
| Silicone premix | 3.24 | 3.24 | 3.24 |
| $AE_3S$ | 47.36 | 47.36 | 47.36 |
| Selenium sulfide premix | 4.00 | 4.00 | 4.00 |
| Methocel premix | -- | -- | 1.30 |
| DRO water | 0.527 | 0.527 | -- |
| Sodium citrate | 0.71 | 0.71 | 0.71 |
| Citric acid | 0.60 | 0.60 | 0.60 |
| Fragrance | 0.65 | 0.65 | 0.65 |
| Preservative | 0.033 | 0.033 | 0.033 |
| | 100.000 | 100.000 | 100.000 |

The above compositions are prepared by heating all of the premixes except for the selenium sulfide premix in separate making containers to 71° C, agitating them and cooling the compositions to 38° C. The selenium sulfide premix and the methocel premix are then mixed together and then combined with the other premixes to form the main mix.

## Claims

**1.** A lotion shampoo composition comprising:
(a) from 10% to 30% by weight of a synthetic surfactant;
(b) from 0.1% to 5.0% of selenium sulfide having an average particle size of less than 25 µm;
(c) from 0.001% to 0.1% of a cellulose polymer, selected from hydroxypropyl methyl cellulose, carboxymethyl cellulose, sodium carboxymethyl cellulose, hydroxyethyl cellulose and mixtures thereof; and
(d) water.

**2.** A shampoo composition according to Claim 1 wherein the surfactant is selected from anionic surfactants, zwitterionic surfactants, amphoteric surfactants and mixtures thereof.

**3.** A shampoo composition according to Claim 2 wherein the surfactant is anionic.

**4.** A shampoo composition according to Claim 3 wherein the anionic surfactant is selected from alkyl sulfate, ethoxylated alkyl sulfates and mixtures thereof.

**5.** A shampoo composition according to Claim 4 which in addition contains a nonvolatile silicone at a level of from 0.1% to 10% by weight.

**6.** A shampoo compostiion according to Claim 5 wherein a suspending agent is also present at a level of 0.1% to 5% by weight.

7. A shampoo composition according to Claim 6 wherin the polymer is hydroxypropyl methyl cellulose.

8. A shampoo composition according to Claim 7 wherein the suspending agent is ethylene glycol distearate.

## Patentansprüche

1. Lotionsshampoozusammensetzung, umfassend:
   (a) 10 Gew.-% bis 30 Gew.-% von einem synthetischen grenzflächenaktiven Mittel;
   (b) 0,1 % bis 5,0 % von Selensulfid mit einer mittleren Teilchengröße von weniger als 25 µm;
   (c) 0,001 % bis 0,1 % von eine Cellulosepolymer, ausgewählt unter Hydroxypropylmethylcellulose, Carboxymethylcellulose, Natriumcarboxymethylcellulose, Hydroxyethylcellulose und Gemischen hievon; und
   (d) Wasser.

2. Shampoozusammensetzung nach Anspruch 1, worin das grenzflächenaktive Mittel unter anionischen grenzflächenaktiven Mitteln, zwitterionischen grenzflächenaktiven Mitteln, amphoteren grenzflächenaktiven Mitteln und Gemischen hievon ausgewählt ist.

3. Shampoozusammensetzung nach Anspruch 2, worin das grenzflächenaktive Mittel anionisch ist.

4. Shampoozusammensetzung nach Anspruch 3, worin das anionische grenzflächenaktive Mittel unter Alkylsulfaten, ethoxylierten Alkylsulfaten und Gemischen hievon ausgewählt ist.

5. Shampoozusammensetzung nach Anspruch 4, welche zusätzlich ein nicht-flüchtiges Silikon in einer Menge von 0,1 Gew.-% bis 10 Gew.-% enthält.

6. Shampoozusammensetzung nach Anspruch 5, worin ein suspendierendes Mittel in einer Menge von 0,1 Gew.-% bis 5 Gew.-% ebenfalls vorhanden ist.

7. Shampoozusammensetzung nach Anspruch 6, worin das Polymer Hydroxypropylmethylcellulose ist.

8. Shampoozusammensetzung nach Anspruch 7, worin das suspendierende Mittel Ethylenglykoldistearat ist.

## Revendications

1. Composition de shampooing en lotion comprenant :
   (a) de 10% à 30% en poids d'un tensioactif synthétique;
   (b) de 0,1% à 5,0% de sulfure de sélénium ayant une granulométrie moyenne inférieure à 25 µm;
   (c) de 0,001% à 0,1% d'un polymère de cellulose choisi parmi l'hydroxypropylméthylcellulose, la carboxyméthylcellulose, la carboxyméthylcellulose sodique, l'hydroxyéthylcellulose et leurs mélanges; et
   (d) de l'eau.

2. Composition de shampooing selon la revendication 1, dans laquelle le tensioactif est choisi parmi les tensioactifs anioniques, les tensioactifs zwittérioniques, les tensioactifs amphotères et leurs mélanges.

3. Composition de shampooing selon la revendication 2, dans laquelle le tensioactif est anionique.

4. Composition de shampooing selon la revendication 3, dans laquelle le tensioactif anionique est choisi parmi les alkylsulfates, les alkylsulfates éthoxylés et leurs mélanges.

5. Composition de shampooing selon la revendication 4, qui contient, en outre, une silicone non volatile en une proportion de 0,1% à 10% en poids.

6. Composition de shampooing selon la revendication 5, dans laquelle un agent de mise en suspension est aussi présent en une proportion de 0,1% à 5% en poids.

**7.** Composition de shampooing selon la revendication 6, dans laquelle le polymère est l'hydroxypropylméthylcellulose.

**8.** Composition de shampooing selon la revendication 7, dans laquelle l'agent de mise en suspension est le distéarate d'éthylèneglycol.